(19) Europäisches Patentamt
European Patent Office
Office européen des brevets

(11) EP 0 856 007 B1

(12) **EUROPÄISCHE PATENTSCHRIFT**

(45) Veröffentlichungstag und Bekanntmachung des Hinweises auf die Patenterteilung:
**21.04.2004 Patentblatt 2004/17**

(21) Anmeldenummer: **96934675.8**

(22) Anmeldetag: **14.10.1996**

(51) Int Cl.[7]: **C07K 14/75**, C07K 7/64, A61K 38/12, G01N 33/68

(86) Internationale Anmeldenummer:
**PCT/EP1996/004462**

(87) Internationale Veröffentlichungsnummer:
**WO 1997/014716 (24.04.1997 Gazette 1997/18)**

(54) **CYCLOPEPTIDDERIVATE**

CYCLOPEPTIDE DERIVATIVES

DERIVES CYCLOPEPTIDIQUES

(84) Benannte Vertragsstaaten:
**AT BE CH DE DK ES FI FR GB GR IE IT LI LU NL PT SE**
Benannte Erstreckungsstaaten:
**LT LV SI**

(30) Priorität: **18.10.1995 DE 19538741**

(43) Veröffentlichungstag der Anmeldung:
**05.08.1998 Patentblatt 1998/32**

(73) Patentinhaber: **MERCK PATENT GmbH**
**64293 Darmstadt (DE)**

(72) Erfinder:
- **JONCZYK, Alfred**
  **D-64295 Darmstadt (DE)**
- **GOODMAN, Simon, Lawrence**
  **D-64287 Darmstadt (DE)**
- **DIEFENBACH, Beate**
  **D-64289 Darmstadt (DE)**
- **SUTTER, Arne**
  **D-64297 Darmstadt (DE)**
- **KESSLER, Horst**
  **D-85748 Garching (DE)**

(56) Entgegenhaltungen:
**DE-A- 4 310 643**

- **TETRAHEDRON LETTERS, Bd. 35, Nr. 31, 1.August 1994, OXFORD GB, Seiten 5547-5550, XP002026922 L S RICHTER: "Peptide-cyclizations on solid support; a fast and efficient route to small cyclopeptides"**
- **CHEMICAL ABSTRACTS, vol. 126, no. 4, 27.Januar 1997 Columbus, Ohio, US; abstract no. 42247, XP002026924 & PEPT.: CHEM. STRUCT. BIOL., PROC. AM. PEPT. SYMP., 14TH, MEETING DATE 1995, P T P KAUMAYA & R HODGES, EDS. MAYFLOWER SCIENTIFIC, KINGSWINFORD, UK , Seiten 202-206, R HAUBNER ET AL.: "RGD plus X: structure/activity investigations on cyclic RGD peptides"**
- **JOURNAL OF THE AMERICAN CHEMICAL SOCIETY, Bd. 118, Nr. 32, 14.August 1996, DC US, Seiten 7461-7472, XP002026923 R HAUBNER ET AL. : "Structural and functional aspects of RGD/containing cyclic pentapeptides as highly potent and selective integrin alphaV-Beta3 antagonists"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Verbindungen der Formel I

$$R^1-Q^1-X-Q^2-R^2 \qquad I$$

worin

$Q^1$, $Q^2$ jeweils unabhängig voneinander fehlt oder $-NH-(CH_2)_n-CO-$,

$R^1$, $R^2$ jeweils unabhängig voneinander fehlt oder cyclo-(Arg-Gly-Asp-Z), wobei Z in der Seitenkette an $Q^1$ oder $Q^2$ oder, falls $Q^1$ und/oder $Q^2$ fehlt, an X gebunden ist, und

wobei mindestens einer der Reste $R^1$ oder $R^2$ immer enthalten sein muß,

X $-CO-R^{18}-CO-$, und falls $R^1-Q^1-$ oder $R^2-Q^2-$ fehlen $R^{10}$, $R^{13}$, $R^{16}$, Het-CO oder einen über eine -CONH-, -COO-, -NH-C(=S)-NH-, -NH-C(=O)-NH-, $-SO_2NH-$oder -NHCO- Bindung verknüpften fluoreszierenden Farbstoffrest,

Z jeweils unabhängig voneinander einen Aminosäurerest oder einen Di-, Tri- oder Tetrapeptidrest, wobei die Aminosäuren unabhängig voneinander ausgewählt sind aus einer Gruppe bestehend aus Ala, Asn, Asp, Arg, Cys, Gln, Glu, Gly, His, Ile, Leu, Lys, Met, Phe, Pro, Ser, Thr, Trp, Tyr, Val oder M,
wobei die genannten Aminosäuren auch zu N-Methyl-, N-Ethyl-, N-Propyl-, N-Benzyl- oder $C_\alpha$-Methylderivaten derivatisiert sein können, und die Aminosäurereste über die α-Amino- und α-Carboxygruppen peptidartig miteinander verknüpft sind, und
wobei M immer enthalten ist,

M $NH(R^8)-CH(R^3)-COOH$,

$R^3$ $-R^5-R^4$, $-R^6-R^4$, $-R^7-R^4$,

$R^4$ OH, $NH_2$, SH oder COOH,

$R^5$ Alkylen mit 1-6 C-Atomen,

$R^6$ Alkylenphenylen mit 7-14 C-Atomen,

$R^7$ Alkylenphenylalkylen mit 8-15 C-Atomen,

$R^8$ H, A oder Alkylenphenyl mit 7-12 C-Atomen,

A Alkyl mit 1-6 C-Atomen,

$R^{10}$ einfach durch COOH, COOA, $SR^{11}$ oder $NR^{12}R^{12'}$ substituiertes Alkanoyl mit 1-18 C-Atomen,

$R^{11}$ Trityl, Pyridyl-2-thio oder Alkyl-thio mit 1-6 C-Atomen,

$R^{12}$ jeweils unabhängig voneinander H, Alkyl mit 1-8 C-Atomen oder eine Aminoschutzgruppe,

$R^{12'}$ Aklyl mit 1-8 C- Atomen oder eine Amino-Schutzgruppe

$R^{13}$ unsubstituiertes oder ein- oder zweifach durch Alkyl mit 1-6 C-Atomen, Alkoxy mit 1-4 C-Atomen, Alkanoyl mit 1-8 C-Atomen, Hal, $SR^{14}$ oder $NR^{15}R^{15'}$ substituiertes Aroyl mit 7-11 C-Atomen,

$R^{14}$ H oder A,

$R^{15}$, $R^{15'}$ jeweils unabhängig voneinander H oder A,

$R^{16}$ unsubstituiertes oder im Arylteil ein-, zwei- oder dreifach durch Hal, Alkoxy mit 1-6 C-Atomen oder OH substituiertes Aralkanoyl mit 7-19 C-Atomen, worin der Arylteil auch eine

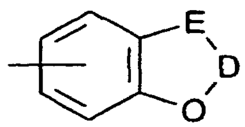

- Gruppe sein kann,

E $CH_2$ oder O,

D Carbonyl oder $[C(R^{17}R^{17'})]_m$,

$R^{17}$, $R^{17'}$ jeweils unabhängig voneinander H oder A,

$R^{18}$ fehit oder
$R^{19}$, $R^{20}$ , $R^{19}$-$R^{20}$-$R^{19}$, unsubstituiertes oder ein- oder zweifach durch $R^5$ substituiertes Phenylen, wobei die Kettenlänge von $R^5$ jeweils unabhängig voneinander ist,

$R^{19}$ Alkylen mit 1-8 C-Atomen, wobei 1 oder 2 Methylengruppen durch S, -CH=CH- oder -C≡C- ersetzt sein können,

$R^{20}$ Cycloalkylen mit 3-7 C-Atomen,

Hal F, Cl, Br oder I,

Het einen ein- oder zweikemigen gesättigten, ungesättigten oder aromatischen Heterocyclus mit 1 bis 4 N-, O- und/ oder S- Atomen, über N oder C gebunden, der unsubstituiert oder ein-, zwei- oder dreifach durch Hal, A, $R^3$, $NR^4R^{4'}$, CN, $NO_2$ und/oder Carbonylsauerstoff substituiert sein kann,

n 1, 2, 3, 4, 5, 6, 7, 8, 9 oder 10

und

m 1 oder 2 bedeuten,

wobei, sofern es sich um Reste optisch aktiver Aminosäuren und Aminosäurederivate handelt, sowohl die D- als auch die L-Formen eingeschlossen sind,
sowie deren Salze.

Ähnliche Verbindungen sind aus der DE 43 10 643 bekannt. Die dort beschriebenen cyclischen Pentapeptide weisen jedoch einen geringeren Substitutionsgrad auf.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der $\alpha_V$-, $\beta_3$- oder $\beta_5$-Integrin-Rezeptoren mit Liganden hemmen, wie z. B. die Bindung von Fibrinogen an den $\beta_3$-Integrinrezeptor. Besondere Wirksamkeit zeigen die Verbindungen im Fall der Integrine $\alpha_V\beta_3$, $\alpha_V\beta_5$, $\alpha_{IIb}\beta_3$ sowie $\alpha_V\beta_1$, $\alpha_V\beta_6$ und $\alpha_V\beta_8$.
Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Verbindungen der Formel I, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:

Die Verbindungen können die Bindung von Metallproteinasen an Integrine hemmen und so verhindern, daß die Zellen die enzymatische Aktivität der Proteinase nutzen können. Ein Beispiel ist in der Hemmbarkeit der Bindung von MMP-2- (Matrix-Metallo-Proteinase-2-) an den Vitronektin-Rezeptor $\alpha_v\beta_3$ durch ein Cyclo-RGD-Peptid zu finden, wie in P.C. Brooks et al., Cell 85, 683-693 (1996) beschrieben.

Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Die Verbindungen der Formel I können als Arzneimittelwirkstoffe in der Human- und Veterinärmedizin eingesetzt werden, insbesondere zur Prophylaxe und/oder Therapie von Thrombose, myocardialem Infarkt, Arteriosklerose, Entzündungen, Apoplexie, Angina pectoris, Tumorerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, viraler Infektion, bakterieller Infektion, Pilzinfektion, bei akutem Nierenversagen und bei der Wundheilung zur Unterstützung der Heilungsprozesse.

Die Verbindungen der Formel I können als antimikrobiell wirkende Substanzen bei Operationen eingesetzt werden, wo Biomaterialien, Implantate, Katheter oder Herzschrittmacher verwendet werden.
Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Die vor- und nachstehend aufgeführten Abkürzungen von Aminosäureresten stehen für die Reste folgender Aminosäuren:

| | |
|---|---|
| Abu | 4-Aminobuttersäure |
| Aha | 6-Aminohexansäure, 6-Aminocapronsäure |
| Ala | Alanin |
| Asn | Asparagin |
| Asp | Asparaginsäure |
| Arg | Arginin |
| Cys | Cystein |
| Dab | 2,4-Diaminobuttersäure |
| Dap | 2,3-Diaminopropionsäure |
| Gln | Glutamin |
| Glp | Pyroglutaminsäure |
| Glu | Glutaminsäure |
| Gly | Glycin |
| His | Histidin |
| homo-Phe | homo-Phenylalanin |
| Ile | Isoleucin |
| Leu | Leucin |
| Lys | Lysin |
| Met | Methionin |
| Nle | Norleucin |
| Orn | Omithin |
| Phe | Phenylalanin |
| Phg | Phenylglycin |

(fortgesetzt)

| | |
|---|---|
| 4-Hal-Phe | 4-Halogen-phenylalanin |
| Pro | Prolin |
| Ser | Serin |
| Thr | Threonin |
| Trp | Tryptophan |
| Tyr | Tyrosin |
| Val | Valin. |

Ferner bedeuten nachstehend:

| | |
|---|---|
| Ac | Acetyl |
| BOC | tert.-eutoxycaubonyl |
| CBZ oder Z | Benzyloxycarbonyl |
| DCCI | Dicyclohexylcarbodiimid |
| DMF | Dimethylformamid |
| EDCI | N-Ethyl-N,N'-(dimethylaminopropyl)-carbodiimid |
| Et | Ethyl |
| FCA | Fluoresceincarbonsäure |
| FITC | Fluoresoeinisothiocyanat |
| Fmoc | 9-Fluorenylmethoxycarbonyl |
| FTH | Fluoresceinthiohamstoff |
| HOBt | 1-Hydroxybenzotriazol |
| Me | Methyl |
| MBHA | 4-Methyl-benzhydrylamin |
| Mtr | 4-Methoxy-2,3,6-trimethylphenyl-sulfonyl |
| HONSu | N-Hydroxysuccinimid |
| OBut | tert.-Butylester |
| Oct | Octanoyl |
| OMe | Methylester |
| OEt | Ethylester |
| POA | Phenoxyacetyl |
| Sal | Salicyloyl |
| TFA | Trifluoressigsäure |
| Trt | Trityl (Triphenylmethyl). |

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschossen. Ferner können die Aminosäuren, z. B. als Bestandteil von Verbindungen der Formel I, mit entsprechenden an sich bekannten Schutzgruppen versehen sein.

In die erfindungsgemäßen Verbindungen sind auch sogenannte Prodrug-Derivate eingeschossen, d. h. mit z. B. Alkyl- oder Acylgruppen, Zuckem oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung ist ferner ein Verfahren zur Herstellung von Verbindungen der Formel I nach Anspruch 1 sowie ihrer Salze, dadurch gekennzeichnet, daß man

(a) eine Verbindung der Formel II

$$H\text{-}Q^1\text{-}R^1 \qquad II$$

worin
$Q^1$ und $R^1$ die in Anspruch 1 angegebene Bedeutung haben, in einer Acylierungsreaktion mit einer Verbindung der Formel III

$$X\text{-}L \qquad \text{III}$$

worin
X die in Anspruch 1 angegebene Bedeutung hat, und

L Cl, Br, I oder eine freie oder reaktionsfähig funktionell abgewandelte OH-Gruppe bedeutet, umsetzt, oder

b) daß man eine Verbindung der Formel IV

$$H\text{-}Q^2\text{-}R^2 \qquad \text{IV}$$

worin
$Q^2$ und $R^2$ die in Anspruch 1 angegebene Bedeutung hat, in einer Acylierungsreaktion mit einer Verbindung der Formel V

$$R^1\text{-}Q^1\text{-}X\text{-}L \qquad \text{V}$$

worin $R^1$, $Q^1$, X und L die angegebene Bedeutung haben, umsetzt, oder

c) daß man eine Verbindung der Formel II

$$H\text{-}Q^1\text{-}R^1 \qquad \text{II}$$

worin
$Q^1$ und $R^1$ die in Anspruch 1 angegebene Bedeutung haben,
in einer Additionsreaktion mit einer Verbindung der Formel VI

$$X\text{-}U \qquad \text{VI}$$

worin

X die in Anspruch 1 angegebene Bedeutung hat und
U -N=C=O, -N=C=S oder Maleinimidyl bedeutet, umsetzt,

oder

d) daß man sie aus einem ihrer funktionellen Derivate durch Behandeln mit einem sotvolysierenden oder hydrogenolysierenden Mittel in Freiheit setzt,

und/oder daß man eine basische oder saure Verbindung der Formel I durch Behandeln mit einer Säure oder Base in eines ihrer Salze überführt.

Vor- und nachstehend haben die Reste $Q^1$, $Q^2$, $R^1$, $R^2$, X und L die bei den Formeln I, II und III angegebenen Bedeutungen, sofern nicht ausdrücklich etwas anderes angegeben ist.

In den vorstehenden Formeln steht Alkyl vorzugsweise für Methyl, Ethyl, Propyl, Isopropyl, Butyl, Isobutyl, sek.-Butyl oder tert.-Butyl, femer auch für Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Alkylen bedeutet bevorzugt Methylen, Ethylen, Propylen, Butylen, Pentylen oder Hexylen.

Alkylenphenyl ist vorzugsweise Benzyl oder Phenethyl. Alkylenphenylalkylen bedeutet vorzugsweise 4-Methylenbenzyl oder 4-Ethylenbenzyl.

$Q^1$ und $Q^2$ bedeuten bevorzugt jeweils unabhängig voneinander 6-Aminohexansäure (6-Aminocapronsäure) oder fehlen, wobei vorzugsweise z. B. $Q^1$ 6-Aminohexansäure bedeutet und $Q^2$ fehlt.

M bedeutet vorzugsweise Dap, Ser, Cys, Asp, D-Asp, Dab, Homoserin, Homocystein, Glu, D-Glu, Thr, Om, Lys, D-Lys, 4-Aminomethyl-Phe oder 4-Aminomethyl-D-Phe.

Die in den Bedeutungen für Z genannten Aminosäuren und Aminosäurereste können auch derivatisiert sein, wobei die N-Methyl-, N-Ethyl-, N-Propyl-, N-Benzyl- oder $C_\alpha$-Methylderivate bevorzugt sind.
Weiter bevorzugt sind Derivate von Asp und Glu, insbesondere die Methyl-, Ethyl, Propyl, Butyl, tert.-Butyl, Neopentyl- oder Benzylester der Seitenketten-carboxy-gruppen, ferner auch Derivate von Arg, das an der $-NH-C(=NH)-NH_2$ -Gruppe mit einem Acetyl-, Benzoyl-, Methoxycarbonyloder Ethoxycarbonylrest substituiert sein kann.

Z bedeutet vorzugsweise M, weiter bevorzugt D-Phe-M, D-Trp-M, D-Tyr-M, D-Phe-Lys, D-Phe-D-Lys, D-Trp-Lys, D-Trp-D-Lys, D-Tyr-Lys, D-Tyr-D-Lys, D-Phe-Orn, D-Phe-Dab, D-Phe-Dap, D-Phe-D-Orn, D-Phe-D-Dab, D-Phe-D-Dap, D-Phe-4-Aminomethyl-Phe, D-Phe-4-Aminomethyl-D-Phe, D-Trp-4-Aminomethyl-Phe, D-Trp-4-Aminomethyl-D-Phe, D-Tyr-4-Aminomethyl-Phe, D-Tyr-4-Aminomethyl-D-Phe, D-Phe-Asp, D-Phe-D-Asp, D-Trp-Asp, D-Trp-D-Asp, D-Tyr-Asp, D-Tyr-D-Asp, D-Phe-Cys, D-Phe-D-Cys, D-Trp-Cys, D-Trp-D-Cys, D-Tyr-Cys, D-Tyr-D-Cys, Phe-D-Lys, Trp-D-Lys, Tyr-D-Lys, Phe-Orn, Phe-Dab, Phe-Dap, Trp-Orn, Trp-Dab, Trp-Dap, Tyr-Orn, Tyr-Dab, Tyr-Dap, Phe-4-Aminomethyl-D-Phe, Trp-4-Aminomethyl-D-Phe, Tyr-4-Aminomethyl-D-Phe, Phe-D-Asp, Trp-D-Asp, Tyr-D-Asp, Phe-D-Cys, Trp-D-Cys, Tyr-D-Cys, D-Phe-Lys-Gly, D-Phe-M-Gly, D-Trp-Lys-Gly, D-Trp-M-Gly, D-Tyr-Lys-Gly, D-Tyr-M-Gly, D-Phe-Val-Lys, D-Phe-Gly-Lys, D-Phe-Ala-Lys, D-Phe-Ile-Lys, D-Phe-Leu-Lys, D-Trp-Val-Lys, D-Trp-Gly-Lys, D-Trp-Ala-Lys, D-Trp-Ile-Lys, D-Trp-Leu-Lys, D-Tyr-Val-Lys, D-Tyr-Gly-Lys, D-Tyr-Ala-Lys, D-Tyr-Ile-Lys, D-Tyr-Leu-Lys, ferner auch M-Pro-Ala-Ser-Ser.

Der Rest $-R^6-R^4$ bedeutet bevorzugt 2-, 3- oder 4-Hydroxybenzyl, 2-, 3-oder 4-Aminobenzyl, 2-, 3- oder 4-Mercaptobenzyl, 2-, 3- oder 4- Carboxybenzyl, ferner bevorzugt 2-, 3- oder 4-Hydroxyphenethyl, 2-,3- oder 4-Aminophenethyl, 2-, 3- oder 4- Mercaptophenethyl, 2-, 3- oder 4-Carboxyphenethyl.

Alkanoyl bedeutet vorzugsweise Formyl, Acetyl, Propionyl, Butyryl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl oder Octadecanoyl.

Aroyl bedeutet vorzugsweise Benzoyl oder Naphthoyl.

$R^{13}$ ist unsubstituiertes, vorzugsweise - wie angegeben - monosubstituiertes Benzoyl, im einzelnen bevorzugt Benzoyl, o-, m- oder p-Methylbenzoyl, o-, m- oder p-Ethylbenzoyl, o-, m- oder p-Propylbenzoyl, o-, m-oder p-Isopropylbenzoyl, o-, m- oder p-tert.-Butylbenzoyl, o-, m- oder p-Aminobenzoyl, o-, m- oder p-(N-Methylamino)-benzoyl, o-, m- oder p-Methoxybenzoyl, o-, m- oder p-Ethoxybenzoyl, o-, m- oder p-(N,N-Dimethylamino)-benzoyl, o-, m- oder p-(N-Ethylamino)-benzoyl, o-, m- oder p-(N,N-Diethylamino)-benzoyl, o-, m- oder p-Fluorbenzoyl, o-, m- oder p-Brombenzoyl, o-, m- oder p- Chlorbenzoyl, o-, m- oder p-Formylbenzoyl, o-, m- oder p-Acetylbenzoyl, o-, m- oder p-Propionylbenzoyl, o-, m- oder p-Butyrylbenzoyl, o-, m- oder p-Pentanoylbenzoyl, o-, m- oder p-Methylthiobenzoyl, weiter bevorzugt 2,3-, 2,4-, 2,5-, 2,6-, 3,4- oder 3,5-Difluorbenzoyl, 2,3-, 2,4-, 2,5-, 2,6-, 3,4- oder 3,5-Dichlorbenzoyl, 2,3-, 2,4-, 2,5-, 2,6-, 3,4- oder 3,5-Dibrombenzoyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyloder 3-Methyl-4-chlorbenzoyl, 2-Brom-3-methyl-, 2-Brom-4-Methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyloder 3-Methyl-4-brombenzoyl, 2,5- oder 3,4-Dimethoxybenzoyl.

$R^{16}$ ist unsubstituiertes, vorzugsweise - wie angegeben - monosubstituiertes Phenylacetyl, im einzelnen bevorzugt Phenylacetyl, o-, m- oder p-Methoxyphenylacetyl, o-, m- oder p-Hydroxyphenylacetyl, o-, m- oder p-Ethoxyphenylacetyl, o-, m- oder p-Fluorphenylacetyl, o-, m- oder p-Bromphenylacetyl, o-, m- oder p- Chlorphenylacetyl, weiter bevorzugt 3-Phenylpropionyl, 4-Phenylbutyryl, 5-Phenylpentanoyl, 6-Phenylhexanoyl, 7-Phenylheptanoyl, 8-Phenytoctanoyl, 9-Phenylnonanoyl, 10-Phenyldecanoyl, 11-Phenylundecanoyl, 12-Phenyldodecanoyl oder 13-Phenyltridecanoyl, ferner 2,3-Methylendioxyphenyl, 3,4-Methylendioxyphenyl, 2,3-Dihydrobenzofuranyl oder 2,3-Dihydro-2-oxobenzofuranyl.

Cycloalkylen bedeutet bevorzugt Cyclopropylen. 1.2- oder 1,3-Cyclobutylen, 1,2- oder 1,3-Cyclopentylen, 1,2-, 1,3- oder 1,4-Cyclohexylen, ferner 1,2- , 1,3- oder 1,4-Cycloheptylen.

D bedeutet vorzugsweise $CH_2$, ebenso ist auch Carbonyl bevorzugt.

Het ist vorzugsweise 2- oder 3-Furyl, 2- oder 3-Thienyl, 1-, 2- oder 3-Pyrrolyl, 1-, 2, 4- oder 5-Imidazotyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- , oder 8-Cinnolinyl, 2-, 4-, 5-, 6-, 7- oder 8-Chinazolinyl. Die heterocyclischen Reste können auch teilweise oder vollständig hydriert sein. Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3-oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2-oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl.

Aminoschutzgruppe bedeutet vorzugsweise Acetyl, Propionyl, Butyryl, Phenylacetyl, Benzoyl, Toluyl, POA, Methoxycarbonyl, Ethoxycarbonyl, 2,2,2-Trichlorethoxycarbonyl, BOC, 2-Iodethoxycarbonyl, CBZ ("Carbobenzoxy"), 4-Methoxybenzyloxycarbonyl, FMOC, Mtr oder Benzyl.

Fluoreszierender Farbstoffrest bedeutet vorzugsweise 7-Acetoxycoumarin-3-yl, Fluorescein-5-(und/oder 6-)yl, 2',7'-Dichlorfluorescein-5-(und 6-)yl, Dihydrotetramethylrosamin-4-yl, Tetramethylrhodamin-5-(und/oder 6-)yl, 4,4-Difluor-5,7-dimethyl-4-bora-3a,4a-diaza-s-indacen-3-ethyl oder 4,4-Difluor-5,7-diphenyl-4-bora-3a,4a-diaza-s-indacen-3-ethyl.

Geeignete funktionalisierte fluoreszierende Farbstoffreste, die als Reagenzien zur Herstellung der erfindungsgemäßen Verbindungen der Formel I dienen können, sind z. B. beschrieben in "Handbook of Fluorescent Probes and Research Chemicals, 5th Edition, 1992-1994, by R. P. Haughland, Molecular Probes, Inc.".

m ist vorzugsweise 1, ferner bevorzugt 2.

Hal bedeutet vorzugsweise F, Cl oder Br, aber auch I.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen und worin die nicht näher bezeichneten Reste die bei der Formel I angegebene Bedeutung haben, worin jedoch

in a)

| | |
|---|---|
| $Q^1$ und $Q^2$ | fehlen, |
| $R^1$ und $R^2$ | Cyclo-(Arg-Gly-Asp-D-Phe-Lys) und |
| X | $-CO-(CH_2)_n-CO-$ bedeuten; |

in b)

| | |
|---|---|
| $Q^2$ und $R^2$ | fehlen, |
| $Q^1$ | $-NH-(CH_2)_5-CO-$, |
| $R^1$ | Cyclo-(Arg-Gly-Asp-Z) und |
| X bedeuten; | einen fluoreszierenden Farbstoffrest |

in c)

| | |
|---|---|
| $Q^2$ und $R^2$ | fehlen, |
| $Q^1$ | $-NH-(CH_2)_5-CO-$, |
| $R^1$ | Cyclo-(Arg-Gly-Asp-M) und |
| X | Fluoreszeinoyl bedeuten; |

in d)

| | |
|---|---|
| $Q^1$ und $Q^2$ | fehlen, |
| $R^1$ und $R^2$ | Cyclo-(Arg-Gly-Asp-M) und |
| X | $-CO-(CH_2)_8-CO-$ bedeuten; |

in e)

| | |
|---|---|
| $Q^1$, $Q^2$ und $R^2$ | fehlen, |
| $R^1$ | Cyclo-(Arg-Gly-Asp-Z) und |
| X | $CH_3$-$(CH_2)_{16}$-CO- bedeuten. |

Besonders bevorzugt sind Verbindungen der Formel VII

Cyclo-(Arg-Gly-Asp-D-Phe-Lys($Q^1$-X)) VII,

worin $Q^1$ die in Anspruch 1 angegebene Bedeutung hat,
und wobei $Q^1$ an die Seitenkette des Lysins gebunden ist, oder falls $Q^1$ fehlt, X an die Seitenkette des Lysins gebunden ist,
und worin X vorzugsweise
einfach durch COOH, COOA, $SR^{14}$ oder $NR^{15}R^{15'}$ substituiertes Alkanoyl mit 1-18 C-Atomen, FCA oder FTH,
unsubstituiertes oder ein- oder zweifach durch Alkyl mit 1-6 C-Atomen, Alkoxy mit 1-4 C-Atomen, Alkanoyl mit 1-8 C-Atomen, Hal, $SR^{14}$ oder $NR^{15}R^{15'}$ substituiertes Aroyl mit 7-11 C-Atomen, wobei $R^{14}$, $R^{15}$ und $R^{15'}$ die in Anspruch 1 angegebenen Bedeutungen haben,
bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit Verbindungen der Formel III umsetzt.

Die Verbindungen der Formel II und III sind in der Regel bekannt. Sind sie nicht bekannt, so können sie nach an sich bekannten Methoden hergestellt werden.

In den Verbindungen der Formel III bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

L bedeutet vorzugsweise H, F, Cl, Br oder -ON-Succinimid.

Die Umsetzung erfolgt in der Regel in einem inerten Lösungsmittel, in Gegenwart eines säurebindenden Mittels vorzugsweise einer organischen Base wie Triethylamin, Dimethylanilin, Pyridin oder Chinolin oder eines Überschusses der Carboxykomponente der Formel III.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Verbindungen der Formel I können weiterhin erhalten werden, indem man Verbindungen der Formel IV mit Verbindungen der Formel V umsetzt.
Die Ausgangsverbindungen der Formel IV und V sind in der Regel bekannt. Sind sie nicht bekannt, so können sie

nach an sich bekannten Methoden hergestellt werden.

In den Verbindungen der Formel V bedeutet der Rest L vorzugsweise eine voraktivierte Carbonsäure, vorzugsweise ein Carbonsäurehalogenid, symmetrisches oder gemischtes Anhydrid oder einen Aktivester. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
L bedeutet vorzugsweise F, Cl, Br oder -ON-Succinimid.

Die Umsetzung der Verbindungen der Formel IV mit Verbindungen der Formel V erfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Verbindungen der Formel I können weiterhin erhalten werden, indem man Verbindungen der Formel II mit Verbindungen der Formel VI umsetzt. Die Ausgangsverbindungen der Formel II und VI sind in der Regel bekannt. Sind sie nicht bekannt, so können sie nach an sich bekannten Methoden hergestellt werden.
Die Umsetzung von Verbindungen der Formel II mit Verbindungen der Formel III stellt eine typische Addition an Isothiocyanate dar. Beschrieben sind derartige Additionen in der Literatur (z.B. in den Standardwerken wie Houben-Weyl, Methoden der organischen Chemie, Georg-Thieme-Verlag, Stuttgart;).

Cyclische Verbindungen der Formel $R^1$ und/oder $R^2$ können durch Cyclisierung der linearen Verbindungen hergestellt werden, wie z. B. in DE 43 10 643 oder in Houben-Weyl, I.c., Band 15/II, Seiten 1 bis 806 (1974) beschrieben.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer $NH_2$-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"Ophenylgruppe enthalten (worin R" eine Hydroxyschutzgruppe bedeutet).

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyloder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Iodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sutfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure. Ether wie Tetrahydrofuran oder Dioxan, Amide wie DMF, halogenierte

Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0° und 50°, vorzugsweise arbeitet man zwischen 15 und 30° oder Raumtemperatur.

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt.

Hydrogenolytisch entfembare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluotsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyloder Diisopropyl-ammoniumsalze, Monoethyl-, Diethyl- oder Diisopropylammoniumsalze, Cyclohexyl-, Dicyclohexylammoniumsalze, Dibenzylethylendiammoniumsalze, weiterhin z. B. Salze mit Arginin oder Lysin.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungsund/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine. Für die Applikation als Inhalationsspray können Sprays verwendet werden, die den Wirkstoff entweder gelöst oder suspendiert in einem Treibgas oder Treibgasgemisch (z. B. $CO_2$ oder Fluorchlorkohlenwasserstoffen) ent-

halten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die Verbindungen der Formel I und ihre physiologisch unbedenklichen Salze können als Integrininhibitoren bei der Bekämpfung von Krankheiten, insbesondere von pathologisch angiogenen Erkrankungen, Thrombosen, Herzinfarkt, koronaren Herzerkrankungen, Arteriosklerose, Tumoren, Osteoporose, Entzündungen und Infektionen verwendet werden.

Dabei können die erfindungsgemäßen Substanzen in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die neuen Verbindungen der Formel I in der analytischen Biologie und Molekularbiologie verwendet werden.

Die neuen Verbindungen der Formel I, wobei X einen über eine -CONH-, -COO-, -NH-C(=S)-NH-, -NH-C(=O)-NH-, $-SO_2NH-$ oder -NHCO- Bindung verknüpften fluoreszierenden Farbstoffrest bedeutet, können als diagnostische Marker in der FACS (Fluorescence Activated Cell Sorter)-Analyse und Fluoreszenz-Mikroskopie verwendet werden.

Der Einsatz von gelabelten Verbindungen in der Fluoreszenz-Mikroskopie ist z. B. beschrieben von Y.-L. Wang und D. L. Taylor in "Fluorescence Microscopy of Living Cells in Culture, Part A + B, Academic Press, Inc. 1989".

Die neuen Verbindungen der Formel I können auch in der Affinitätschromatographie zum Eluieren von gebundenen Proteinen verwendet werden.

Insbesondere können sie als Integrinliganden zum Eluieren von Integrinen verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

RZ = Retentionszeit (Minuten) bei HPLC in den folgenden Systemen:

[A]

| | |
|---|---|
| Säule | Nucleosil 7C 18 250 x 4 mm |
| Eluent A | 0,1 % TFA in Wasser |
| Eluent B | 0,1 % TFA in Acetonitril |
| Fluß | 1 ml/min |
| Gradient | 20 - 50 % B / 30 min. |

[B]

50 minütiger Gradient von 0-80 % 2-Propanol in Wasser mit 0,3 % TFA bei 1 ml/min auf einer Säule Lichrosorb$^R$ RP Select B (7 µm) 250 x 4 mm

[C]

| | |
|---|---|
| Säule | Lichrospher (5 µm) 100RP8 125 x 4 mm |
| Eluent A | 0,01 M Na-Phosphat pH 7.0 |
| Eluent B | 0,005 M Na-Phosphat pH 7.0 / 60 Vol. % 2-Propanol |
| Fluß | 0,7 ml/min |
| Gradient | 1 - 99 % B / 50 min. |

Massenspektrometrie (MS): EI (Elektronenstoß-Ionisation) $M^+$

FAB (Fast Atom Bombardment) $(M+H)^+$

Beispiel 1

Zu einer Lösung aus 3,05 g Cyclo-(Arg-Gly-Asp-D-Phe-Lys) [erhältlich durch Cyclisierung von H-Arg(Mtr)-Gly-Asp(OBut)-D-Phe-Lys(BOC)-OH zu Cyclo-(Arg(Mtr)-Gly-Asp(OBut)-D-Phe-Lys(BOC)) und anschließender Abspaltung der Schutzgruppen] in 100 ml DMF gibt man 1,0 g O-Acetylsalicylsäure-N-succinimidylester [erhältlich durch Reaktion von Acetylsalicylsäure mit HONSu in Essigester, DMF und 1,2 Äquivalenten Diisopropylcarbodiimid, FAB 278] und 0,5 g Triethylamin.
Man rührt 5 Stunden bei Raumtemperatur und erhält nach üblicher Aufarbeitung, unter gleichzeitiger Abspaltung der Acetylgruppe, Cyclo-(Arg-Gly-Asp-D-Phe-Lys($N^{\varepsilon}$-Sal)) x TFA; RZ [B] 22.0; FAB 724.
Analog erhält man durch Umsetzung von Cyclo-(Arg-Gly-Asp-D-Phe-Lys)

mit Phenylpropionsäure-N-succinimidylester(PhEtCO-ONSu) Cyclo-(Arg-Gly-Asp-D-Phe-Lys($N^{\varepsilon}$-PhEtCO)) x TFA; RZ [C] 28.2; FAB 736;

mit 3,3,3-Tris-(4-chlorphenyl)-propionsäure-N-succinimidylester (TCPP-ONSu)
Cyclo-(Arg-Gly-Asp-D-Phe-Lys($N^{\varepsilon}$-TCPP)) x TFA; RZ [B] 33.19; FAB 992;

mit S-Tritylmercaptopropionsäure-N-succinimidylester (TrtSEtCO-ONSu)
Cyclo-(Arg-Gly-Asp-D-Phe-Lys($N^{\varepsilon}$-TrtSEtCO)) x TFA; RZ [B] 33.4; FAB 934;

mit Benzyloxycarbonylchlorid (CBZ-Cl)
Cyclo-(Arg-Gly-Asp-D-Phe-Lys($N^{\varepsilon}$-CBZ));

mit Octanoylanhydrid
Cyclo-(Arg-Gly-Asp-D-Phe-Lys($N^{\varepsilon}$-Oct)) x TFA; RZ [B] 27.58; FAB 730;

mit FCA-N-succinimidylester
Cyclo-(Arg-Gly-Asp-D-Phe-Lys($N^{\varepsilon}$-FCA)); RZ [B] 24.18; FAB 962;

mit FITC
Cyclo-(Arg-Gly-Asp-D-Phe-Lys($N^{\varepsilon}$-FTH)) x TFA; RZ [B] 27.3; FAB 994,
woraus mit NH4HCO3 das innere Salz erhalten werden kann, RZ [B] 22.26;

Analog erhält man durch Umsetzung von Cyclo-(Arg-Gly-Asp-D-Phe-N(Me)-Lys) mit FITC
Cyclo-(Arg-Gly-Asp-D-Phe-N(Me)-Lys($N^{\varepsilon}$-FTH)); RZ [B] 22.64; FAB 1007;

mit Benzyloxycarbonylchlorid (CBZ-Cl)
Cyclo-(Arg-Gly-Asp-D-Phe-N(Me)-Lys($N^{\varepsilon}$-CBZ)); RZ 23.35; FAB 752.

Beispiel 2

Zu einer Lösung von 3,05 g Cyclo-(Arg-Gly-Asp-D-Phe-Lys) in 40 ml 5 %iger wässriger $NaHCO_3$ und 40 ml THF gibt man 6g BOC-Aha-N-succinimidylester. Man rührt 4 Stunden, arbeitet wie üblich auf und erhält Cyclo-(Arg-Gly-Asp-D-Phe-Lys(BOC-Aha)); RZ [C] 27.7; FAB 817.
Nach Abspaltung der BOC-Gruppe in HCl/Dioxan erhält man nach üblicher Aufarbeitung Cyclo-(Arg-Gly-Asp-D-Phe-Lys($N^{\varepsilon}$-Aha)) x 2 TFA; RZ [C] 14.76; FAB 717.
Analog zu Beispiel 1 erhält man durch anschließende Umsetzung mit FCA-N-succinimidylester Cyclo-(Arg-Gly-Asp-D-Phe-Lys($N^{\varepsilon}$-FCA-Aha)) x TFA; RZ [B] 23.8; FAB 1075.
Analog erhält man durch Umsetzung von
Cyclo-(Arg-Gly-Asp-D-Phe-Lys($N^{\varepsilon}$-Aha))

mit FITC
Cyclo-(Arg-Gly-Asp-D-Phe-Lys($N^{\varepsilon}$-FTH-Aha))
mit Acetanhydrid
Cyclo-(Arg-Gly-Asp-D-Phe-Lys($N^{\varepsilon}$-Ac-Aha)) x TFA; RZ [B] 17.1; FAB 759;

Beispiel 3

Analog Beispiel 2 erhält man aus Cyclo-(Arg-Gly-Asp-D-Phe-Lys-Gly) [erhältlich durch Cyclisierung von H-Arg (Mtr)-Gly-Asp(OBut)-D-Phe-Lys(BOC)-Gly-OH zu Cyclo-(Arg(Mtr)-Gly-Asp(OBut)-D-Phe-Lys(BOC)-Gly) und anschließender Abspaltung der Schutzgruppen] und Boc-Aha-N-succinimidylester Cyclo-(Arg-Gly-Asp-D-Phe-Lys ($N^{\varepsilon}$-BOC-Aha)-Gly);
Nach Abspaltung der BOC-Gruppe in HCl/Dioxan erhält man nach üblicher Aufarbeitung Cyclo-(Arg-Gly-Asp-D-Phe-Lys($N^{\varepsilon}$-Aha)-Gly) x 2 TFA.

Analog Beispiel 1 erhält man durch anschließende Umsetzung von Cyclo-(Arg-Gly-Asp-D-Phe-Lys($N^{\varepsilon}$-Aha) -Gly) x 2 TFA mit Phenylpropionsäure-N-succinimidylester
Cyclo-(Arg-Gly-Asp-D-Phe-Lys($N^{\varepsilon}$-PhEtCO-Aha)-Gly).

Analog erhält man durch Umsetzung von
Cyclo-(Arg-Gly-Asp-D-Phe-Lys($N^{\varepsilon}$-Aha)-Gly)

mit Octanoylanhydrid
Cyclo-(Arg-Gly-Asp-D-Phe-Lys($N^{\varepsilon}$-Oct-Aha)-Gly)

mit FCA-N-succinimidylester
Cyclo-(Arg-Gly-Asp-D-Phe-Lys($N^{\varepsilon}$-FCA-Aha)-Gly)

mit FITC
Cyclo-(Arg-Gly-Asp-D-Phe-Lys($N^{\varepsilon}$-FTH-Aha)-Gly).

Analog Beispiel 2 erhält man aus Cyclo-(Arg-Gly-Asp-D-Phe-Val-Lys) [erhältlich durch Cyclisierung von H-Arg(Mtr)-Gly-Asp(OBut)-D-Phe-Val-Lys(BOC)-OH zu Cyclo-(Arg(Mtr)-Gly-Asp(OBut)-D-Phe-Val-Lys(BOC)) und anschließender Abspaltung der Schutzgruppen] und Boc-Aha-N-succinimidylester Cyclo-(Arg-Gly-Asp-D-Phe-Val-Lys ($N^{\varepsilon}$-BOC-Aha)) x TFA.
Nach Abspaltung der BOC-Gruppe in HCl/Dioxan erhält man nach üblicher Aufarbeitung Cyclo-(Arg-Gly-Asp-D-Phe-Val-Lys($N^{\varepsilon}$-Aha)) x 2 TFA.

Analog Beispiel 1 erhält man durch anschließende Umsetzung von Cyclo-(Arg-Gly-Asp-D-Phe-Val-Lys($N^{\varepsilon}$-Aha)) x 2 TFA mit Phenylpropionsäure-N-succinimidylester
Cyclo-(Arg-Gly-Asp-D-Phe-Val-Lys($N^{\varepsilon}$-PhEtCO-Aha)).

Beispiel 4

Analog Beispiel 2 erhält man durch Umsetzung von BOC-Aminocapronsäure-N-succinimidylester und den nachstehenden cyclischen Verbindungen

Cyclo-(Arg-Gly-Asp-D-Trp-Lys)
Cyclo-(Arg-Gly-Asp-D-Tyr-Lys)
Cyclo-(Arg-Gly-Asp-D-Phe-D-Lys)
Cyclo-(Arg-Gly-Asp-D-Phe-Cys)
Cyclo-(Arg-Gly-Asp-D-Phe-Dab)
Cyclo-(Arg-Gly-Asp-D-Trp-D-Cys)
Cyclo-(Arg-Gly-Asp-D-Tyr-D-Cys)
Cyclo-(Arg-Gly-Asp-Phe-D-Lys)
Cyclo-(Arg-Gly-Asp-Trp-D-Lys)
Cyclo-(Arg-Gly-Asp-Tyr-D-Lys)
Cyclo-(Arg-Gly-Asp-Phe-D-Cys)
Cyclo-(Arg-Gly-Asp-Phe-Dab)
Cyclo-(Arg-Gly-Asp-Trp-D-Cys)
Cyclo-(Arg-Gly-Asp-Tyr-D-Cys)
Cyclo-(Arg-Gly-Asp-D-Trp-Orn)
Cyclo-(Arg-Gly-Asp-D-Tyr-Orn)
Cyclo-(Arg-Gly-Asp-D-Phe-Orn)
Cyclo-(Arg-Gly-Asp-D-Trp-D-Orn)
Cyclo-(Arg-Gly-Asp-D-Tyr-D-Orn)
Cyclo-(Arg-Gly-Asp-D-Phe-D-Orn)

Cyclo-(Arg-Gly-Asp-D-Trp-Dab)
Cyclo-(Arg-Gly-Asp-D-Tyr-Dab)
Cyclo-(Arg-Gly-Asp-D-Trp-Dap)
Cyclo-(Arg-Gly-Asp-D-Tyr-Dap)
Cyclo-(Arg-Gly-Asp-D-Phe-Dap)
Cyclo-(Arg-Gly-Asp-D-Trp-D-Dap)
Cyclo-(Arg-Gly-Asp-D-Tyr-D-Dap)
Cyclo-(Arg-Gly-Asp-D-Phe-D-Dap)

nachfolgende Peptide

Cyclo-(Arg-Gly-Asp-D-Trp-Lys($N^{\varepsilon}$-BOC-Aha))
Cyclo-(Arg-Gly-Asp-D-Tyr-Lys($N^{\varepsilon}$-BOC-Aha))
Cyclo-(Arg-Gly-Asp-D-Phe-D-Lys($N^{\varepsilon}$-BOC-Aha))
Cyclo-(Arg-Gly-Asp-D-Phe-Cys(S-BOC-Aha))
Cyclo-(Arg-Gly-Asp-D-Phe-Dab($N^{\gamma}$-BOC-Aha))
Cyclo-(Arg-Gly-Asp-D-Trp-D-Cys(S-BOC-Aha))
Cyclo-(Arg-Gly-Asp-D-Tyr-D-Cys(S-BOC-Aha))
Cyclo-(Arg-Gly-Asp-Phe-D-Lys($N^{\varepsilon}$-BOC-Aha))
Cyclo-(Arg-Gly-Asp-Trp-D-Lys($N^{\varepsilon}$-BOC-Aha))
Cyclo-(Arg-Gly-Asp-Tyr-D-Lys($N^{\varepsilon}$-BOC-Aha))
Cyclo-(Arg-Gly-Asp-Phe-D-Cys(S-BOC-Aha))
Cyclo-(Arg-Gly-Asp-Phe-Dab($N^{\gamma}$-BOC-Aha))
Cyclo-(Arg-Gly-Asp-Trp-D-Cys(S-BOC-Aha))
Cyclo-(Arg-Gly-Asp-Tyr-D-Cys(S-BOC-Aha))
Cyclo-(Arg-Gly-Asp-D-Trp-Orn($N^{\delta}$-BOC-Aha))
Cyclo-(Arg-Gly-Asp-D-Tyr-Orn($N^{\delta}$-BOC-Aha))
Cyclo-(Arg-Gly-Asp-D-Phe-Orn($N^{\delta}$-BOC-Aha))
Cyclo-(Arg-Gly-Asp-D-Trp-D-Orn($N^{\delta}$-BOC-Aha))
Cyclo-(Arg-Gly-Asp-D-Tyr-D-Orn($N^{\delta}$-BOC-Aha))
Cyclo-(Arg-Gly-Asp-D-Phe-D-Orn($N^{\delta}$-BOC-Aha))
Cyclo-(Arg-Gly-Asp-D-Trp-Dab($N^{\gamma}$-BOC-Aha))
Cyclo-(Arg-Gly-Asp-D-Tyr-Dab($N^{\gamma}$-BOC-Aha))
Cyclo-(Arg-Gly-Asp-D-Trp-Dap($N^{\beta}$-BOC-Aha))
Cyclo-(Arg-Gly-Asp-D-Tyr-Dap($N^{\beta}$-BOC-Aha))
Cyclo-(Arg-Gly-Asp-D-Phe-Dap($N^{\beta}$-BOC-Aha))
Cyclo-(Arg-Gly-Asp-D-Trp-D-Dap($N^{\beta}$-BOC-Aha))
Cyclo-(Arg-Gly-Asp-D-Tyr-D-Dap($N^{\beta}$-BOC-Aha))
Cyclo-(Arg-Gly-Asp-D-Phe-D-Dap($N^{\beta}$-BOC-Aha)).

Nach Abspaltung der BOC-Gruppe in HCl/Dioxan erhält man die nachstehenden Verbindungen ("A")

Cyclo-(Arg-Gly-Asp-D-Trp-Lys($N^{\varepsilon}$-Aha))
Cyclo-(Arg-Gly-Asp-D-Tyr-Lys($N^{\varepsilon}$-Aha))
Cyclo-(Arg-Gly-Asp-D-Phe-D-Lys($N^{\varepsilon}$-Aha))
Cyclo-(Arg-Gly-Asp-D-Phe-Cys(S-Aha))
Cyclo-(Arg-Gly-Asp-D-Phe-Dab($N^{\gamma}$-Aha))
Cyclo-(Arg-Gly-Asp-D-Trp-D-Cys(S-Aha))
Cyclo-(Arg-Gly-Asp-D-Tyr-D-Cys(S-Aha))
Cyclo-(Arg-Gly-Asp-Phe-D-Lys($N^{\varepsilon}$-Aha))
Cyclo-(Arg-Gly-Asp-Trp-D-Lys($N^{\varepsilon}$-Aha))
Cyclo-(Arg-Gly-Asp-Tyr-D-Lys($N^{\varepsilon}$-Aha))
Cyclo-(Arg-Gly-Asp-Phe-D-Cys(S-Aha))
Cyclo-(Arg-Gly-Asp-Phe-Dab($N^{\gamma}$-Aha))
Cyclo-(Arg-Gly-Asp-Trp-D-Cys(S-Aha))
Cyclo-(Arg-Gly-Asp-Tyr-D-Cys(S-Aha))
Cyclo-(Arg-Gly-Asp-D-Trp-Orn($N^{\delta}$-Aha))
Cyclo-(Arg-Gly-Asp-D-Tyr-Orn($N^{\delta}$-Aha))

Cyclo-(Arg-Gly-Asp-D-Phe-Orn($N^{\delta}$-Aha))
Cyclo-(Arg-Gly-Asp-D-Trp-D-Orn($N^{\delta}$-Aha))
Cyclo-(Arg-Gly-Asp-D-Tyr-D-Orn($N^{\delta}$-Aha))
Cyclo-(Arg-Gly-Asp-D-Phe-D-Orn($N^{\delta}$-Aha))
Cyclo-(Arg-Gly-Asp-D-Trp-Dab($N^{\gamma}$-Aha))
Cyclo-(Arg-Gly-Asp-D-Tyr-Dab($N^{\gamma}$-Aha))
Cyclo-(Arg-Gly-Asp-D-Trp-Dap($N^{\beta}$-Aha))
Cyclo-(Arg-Gly-Asp-D-Tyr-Dap($N^{\beta}$-Aha))
Cyclo-(Arg-Gly-Asp-D-Phe-Dap($N^{\beta}$-Aha))
Cyclo-(Arg-Gly-Asp-D-Trp-D-Dap($N^{\beta}$-Aha))
Cyclo-(Arg-Gly-Asp-D-Tyr-D-Dap($N^{\beta}$-Aha))
Cyclo-(Arg-Gly-Asp-D-Phe-D-Dap($N^{\beta}$-Aha)).

Durch Umsetzung mit Octanoylanhydrid erhält man

Cyclo-(Arg-Gly-Asp-D-Trp-Lys($N^{\varepsilon}$-Oct-Aha))
Cyclo-(Arg-Gly-Asp-D-Tyr-Lys($N^{\varepsilon}$-Oct-Aha))
Cyclo-(Arg-Gly-Asp-D-Phe-D-Lys($N^{\varepsilon}$-Oct-Aha))
Cyclo-(Arg-Gly-Asp-D-Phe-Cys(S-Oct-Aha))
Cyclo-(Arg-Gly-Asp-D-Phe-Dab($N^{\gamma}$-Oct-Aha))
Cyclo-(Arg-Gly-Asp-D-Trp-D-Cys(S-Oct-Aha))
Cyclo-(Arg-Gly-Asp-D-Tyr-D-Cys(S-Oct-Aha))
Cyclo-(Arg-Gly-Asp-Phe-D-Lys($N^{\varepsilon}$-Act-Aha))
Cyclo-(Arg-Gly-Asp-Trp-D-Lys($N^{\varepsilon}$-Oct-Aha))
Cyclo-(Arg-Gly-Asp-Tyr-D-Lys($N^{\varepsilon}$-Oct-Aha))
Cyclo-(Arg-Gly-Asp-Phe-D-Cys(S-Oct-Aha))
Cyclo-(Arg-Gly-Asp-Phe-Dab($N^{\gamma}$-Oct-Aha))
Cyclo-(Arg-Gly-Asp-Trp-D-Cys(S-Oct-Aha))
Cyclo-(Arg-Gly-Asp-Tyr-D-Cys(S-Oct-Aha))
Cyclo-(Arg-Gly-Asp-D-Trp-Orn($N^{\delta}$-Oct-Aha))
Cyclo-(Arg-Gly-Asp-D-Tyr-Orn($N^{\delta}$-Oct-Aha))
Cyclo-(Arg-Gly-Asp-D-Phe-Orn($N^{\delta}$-Oct-Aha))
Cyclo-(Arg-Gly-Asp-D-Trp-D-Orn($N^{\delta}$-Oct-Aha))
Cyclo-(Arg-Gly-Asp-D-Tyr-D-Orn($N^{\delta}$-Oct-Aha))
Cyclo-(Arg-Gly-Asp-D-Phe-D-Orn($N^{\delta}$-Oct-Aha))
Cyclo-(Arg-Gly-Asp-D-Trp-Dab($N^{\gamma}$-Oct-Aha))
Cyclo-(Arg-Gly-Asp-D-Tyr-Dab($N^{\gamma}$-Oct-Aha))
Cyclo-(Arg-Gly-Asp-D-Trp-Dap($N^{\beta}$-Oct-Aha))
Cyclo-(Arg-Gly-Asp-D-Tyr-Dap($N^{\beta}$-Oct-Aha))
Cyclo-(Arg-Gly-Asp-D-Phe-Dap($N^{\beta}$-Oct-Aha))
Cyclo-(Arg-Gly-Asp-D-Trp-D-Dap($N^{\beta}$-Oct-Aha))
Cyclo-(Arg-Gly-Asp-D-Tyr-D-Dap($N^{\beta}$-Oct-Aha))
Cyclo-(Arg-Gly-Asp-D-Phe-D-Dap($N^{\beta}$-Oct-Aha)).

Durch analoge Umsetzung der "A"-Verbindungen mit FCA-N-succinimidylester erhält man

Cyclo-(Arg-Gly-Asp-D-Trp-Lys($N^{\varepsilon}$-FCA-Aha))
Cyclo-(Arg-Gly-Asp-D-Tyr-Lys($N^{\varepsilon}$-FCA-Aha))
Cyclo-(Arg-Gly-Asp-D-Phe-D-Lys($N^{\varepsilon}$-FCA-Aha))
Cyclo-(Arg-Gly-Asp-D-Phe-Cys(S-FCA-Aha))
Cyclo-(Arg-Gly-Asp-D-Phe-Dab($N^{\gamma}$-FCA-Aha))
Cyclo-(Arg-Gly-Asp-D-Trp-D-Cys(S-FCA-Aha))
Cyclo-(Arg-Gly-Asp-D-Tyr-D-Cys(S-FCA-Aha))
Cyclo-(Arg-Gly-Asp-Phe-D-Lys($N^{\varepsilon}$-FCA-Aha))
Cyclo-(Arg-Gly-Asp-Trp-D-Lys($N^{\varepsilon}$-FCA-Aha))
Cyclo-(Arg-Gly-Asp-Tyr-D-Lys($N^{\varepsilon}$-FCA-Aha))
Cyclo-(Arg-Gly-Asp-Phe-D-Cys(S-FCA-Aha))
Cyclo-(Arg-Gly-Asp-Phe-Dab($N^{\gamma}$-FCA-Aha))

Cyclo-(Arg-Gly-Asp-Trp-D-Cys(S-FCA-Aha))
Cyclo-(Arg-Gly-Asp-Tyr-D-Cys(S-FCA-Aha))
Cyclo-(Arg-Gly-Asp-D-Trp-Orn($N^{\delta}$-FCA-Aha))
Cyclo-(Arg-Gly-Asp-D-Tyr-Orn($N^{\delta}$-FCA-Aha))
Cyclo-(Arg-Gly-Asp-D-Phe-Orn($N^{\delta}$-FCA-Aha))
Cyclo-(Arg-Gly-Asp-D-Trp-D-Orn($N^{\delta}$-FCA-Aha))
Cyclo-(Arg-Gly-Asp-D-Tyr-D-Orn($N^{\delta}$-FCA-Aha))
Cyclo-(Arg-Gly-Asp-D-Phe-D-Orn($N^{\delta}$-FCA-Aha))
Cyclo-(Arg-Gly-Asp-D-Trp-Dab($N^{\gamma}$-FCA-Aha))
Cyclo-(Arg-Gly-Asp-D-Tyr-Dab($N^{\gamma}$-FCA-Aha))
Cyclo-(Arg-Gly-Asp-D-Trp-Dap($N^{\beta}$-FCA-Aha))
Cyclo-(Arg-Gly-Asp-D-Tyr-Dap($N^{\beta}$-FCA-Aha))
Cyclo-(Arg-Gly-Asp-D-Phe-Dap($N^{\beta}$-FCA-Aha))
Cyclo-(Arg-Gly-Asp-D-Trp-D-Dap($N^{\beta}$-FCA-Aha))
Cyclo-(Arg-Gly-Asp-D-Tyr-D-Dap($N^{\beta}$-FCA-Aha))
Cyclo-(Arg-Gly-Asp-D-Phe-D-Dap($N^{\beta}$-FCA-Aha)).

Durch analoge Umsetzung der "A"-Verbindungen mit O-Acetylsalicylsäure-N-succinimidylester erhält man

Cyclo-(Arg-Gly-Asp-D-Trp-Lys($N^{\varepsilon}$-Ac-Sal-Aha))
Cyclo-(Arg-Gly-Asp-D-Tyr-Lys($N^{\varepsilon}$-Ac-Sal-Aha))
Cyclo-(Arg-Gly-Asp-D-Phe-D-Lys($N^{\varepsilon}$-Ac-Sal-Aha))
Cyclo-(Arg-Gly-Asp-D-Phe-Cys(S-Ac-Sal-Aha))
Cyclo-(Arg-Gly-Asp-D-Phe-Dab($N^{\gamma}$-Ac-Sal-Aha))
Cyclo-(Arg-Gly-Asp-D-Trp-D-Cys(S-Ac-Sal-Aha))
Cyclo-(Arg-Gly-Asp-D-Tyr-D-Cys(S-Ac-Sal-Aha))
Cyclo-(Arg-Gly-Asp-Phe-D-Lys($N^{\varepsilon}$-Ac-Sal-Aha))
Cyclo-(Arg-Gly-Asp-Trp-D-Lys($N^{\varepsilon}$-Ac-Sal-Aha))
Cyclo-(Arg-Gly-Asp-Tyr-D-Lys($N^{\varepsilon}$-Ac-Sal-Aha))
Cyclo-(Arg-Gly-Asp-Phe-D-Cys(S-Ac-Sal-Aha))
Cyclo-(Arg-Gly-Asp-Phe-Dab($N^{\gamma}$-Ac-Sal-Aha))
Cyclo-(Arg-Gly-Asp-Trp-D-Cys(S-Ac-Sal-Aha))
Cyclo-(Arg-Gly-Asp-Tyr-D-Cys(S-Ac-Sal-Aha))
Cyclo-(Arg-Gly-Asp-D-Trp-Orn($N^{\delta}$-Ac-Sal-Aha))
Cyclo-(Arg-Gly-Asp-D-Tyr-Orn(Nδ-Ac-Sal-Aha))
Cyclo-(Arg-Gly-Asp-D-Phe-Orn($N^{\delta}$-Ac-Sal-Aha))
Cyclo-(Arg-Gly-Asp-D-Trp-D-Orn($N^{\delta}$-Ac-Sal-Aha))
Cyclo-(Arg-Gly-Asp-D-Tyr-D-Orn($N^{\delta}$-Ac-Sal-Aha))
Cyclo-(Arg-Gly-Asp-D-Phe-D-Orn($N^{\delta}$-Ac-Sal-Aha))
Cyclo-(Arg-Gly-Asp-D-Trp-Dab($N^{\gamma}$-Ac-Sal-Aha))
Cyclo-(Arg-Gly-Asp-D-Tyr-Dab($N^{\gamma}$-Ac-Sal-Aha))
Cyclo-(Arg-Gly-Asp-D-Trp-Dap($N^{\beta}$-Ac-Sal-Aha))
Cyclo-(Arg-Gly-Asp-D-Tyr-Dap($N^{\beta}$-Ac-Sal-Aha))
Cyclo-(Arg-Gly-Asp-D-Phe-Dap($N^{\beta}$-Ac-Sal-Aha))
Cyclo-(Arg-Gly-Asp-D-Trp-D-Dap($N^{\beta}$-Ac-Sal-Aha))
Cyclo-(Arg-Gly-Asp-D-Tyr-D-Dap($N^{\beta}$-Ac-Sal-Aha))
Cyclo-(Arg-Gly-Asp-D-Phe-D-Dap($N^{\beta}$-Ac-Sal-Aha)),

wobei gleichzeitig auch die desacetylierten Verbindungen anfallen, die unter üblichen chromatographischen Bedingungen abgetrennt werden.

Beispiel 5

Zu einer Lösung aus 3,05 g Cyclo-(Arg-Gly-Asp-D-Phe-Lys) in 100 ml Dichlormethan gibt man 2,0 g Bernsteinsäure-N-succinimidylestermonomethylester und 0,5 g Triethylamin. Man rührt 5 Stunden bei Raumtemperatur und erhält nach üblicher Aufarbeitung Cyclo-(Arg-Gly-Asp-D-Phe-Lys($N^{\varepsilon}$-$H_3COCO(CH_2)_2CO$)).
Durch Verseifung des Esters mit wässrigem Kaliumhydroxid erhält man Cyclo-(Arg-Gly-Asp-D-Phe-Lys($N^{\varepsilon}$-HOCO $(CH_2)_2CO$)).

Durch anschließende Umsetzung mit HONSu in Essigester erhält man Cyclo-(Arg-Gly-Asp-D-Phe-Lys($N^{\varepsilon}$-SuN-O-CO $(CH_2)_2CO$)).
Analog Beispiel 1 erhält man durch Reaktion mit Cyclo-(Arg-Gly-Asp-D-Phe-Lys-Gly) nachstehendes Cyclo-(Arg-Gly-Asp-D-Phe-Lys($N^{\varepsilon}$-$CO(CH_2)_2CO$-$R^2$)), worin $R^2$ Cyclo-(Arg-Gly-Asp-D-Phe-Lys($N^{\varepsilon}$-)-Gly) bedeutet.

Beispiel 6

Eine Lösung von 1,17 g Cyclo-(Arg(Mtr)-Gly-Asp(OBut)-D-Phe-Lys(CBZ)) in 50 ml Dimethylacetamid wird mit 0,5 ml Essigsäure und 0,5 g Palladium auf Aktivkohle versetzt und 2 Stunden unter Wasserstoffatmosphäre gerührt. Nach Abtrennung des Katalysators und üblicher Aufarbeitung erhält man Cyclo-(Arg(Mtr)-Gly-Asp(OBut)-D-Phe-Lys) ("B"); RZ [A, 30-80 % Acetonitril] 18.6.
Zu einer Lösung von 0,3 g "B" in 15 ml DMF werden 0,075 g Bemsteinsäureanhydrid gegeben und bei Raumtemperatur 12 Stunden gerührt.
Nach üblicher Aufarbeitung erhält man 0,26 g Cyclo-(Arg(Mtr)-Gly-Asp(OBut)-D-Phe-Lys($N^{\varepsilon}$-CO-$(CH_2)_2$-COOH)) ("C"); RZ [A, 30-80 % Acetonitril] 19.2; FAB 972.
Eine Lösung von 0,23 g "B" in 20 ml DMF wird mit 0,1 g EDCI x HCl, 0,075 g HOBt und einer Lösung von 0,3 g "B" in 15 ml DMF versetzt und 12 Stunden gerührt. Nach üblicher Aufarbeitung erhält man Cyclo-(Arg(Mtr)-Gly-Asp (OBut)-D-Phe-Lys)$_2$($COCH_2CH_2CO$) ("D"); RZ [A, 30-80 % Acetonitril] 26.6; FAB 1826.
Eine Lösung bestehend aus 85,5 % TFA, 2 % Wasser, 2,5 % Ethandithiol, 5 % Phenol, 5 % Thioanisol und 0,25 g "D" wird 24 Stunden bei Raumtemperatur gerührt. Nach üblicher Aufarbeitung erhält man Cyclo-(Arg-Gly-Asp-D-Phe-Lys)$_2$ ($COCH_2CH_2CO$); RZ [A, 10-50 % Acetonitril] 20.2; FAB 1289.
Analog erhält man ausgehend von Cyclo-(Arg(Mtr)-Gly-Asp(OBut)-D-Phe-Lys(CBZ)) durch Umsetzung mit Dithiodipropionsäure (DTDP-OH) unter den gleichen Bedingungen wie zuvor das
Bis- $N^{\varepsilon}$-Cyclo-(Arg-Gly-Asp-D-Phe-Lys)-DTDP; RZ 20.73; FAB 1382.
Die nachfolgenden Beispiele betreffen pharmazeutische Zubereitungen:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2 H_2O$, 28,48 g $Na_2HPO_4 \cdot 12 H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Beispiel I: Inhalations-Spray**

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

**Patentansprüche**

**1.** Verbindungen der Formel I

$$R^1\text{-}Q^1\text{-}X\text{-}Q^2\text{-}R^2 \qquad I$$

worin

$Q^1$, $Q^2$ jeweils unabhängig voneinander fehlt oder $-NH-(CH_2)_n-CO-$,

$R^1$, $R^2$ jeweils unabhängig voneinander fehlt oder cyclo-(Arg-Gly-Asp-Z), wobei Z in der Seitenkette an $Q^1$ oder $Q^2$ oder, falls $Q^1$ und/oder $Q^2$ fehlt, an X gebunden ist, und

wobei mindestens einer der Reste $R^1$ oder $R^2$ immer enthalten sein muß,

X -CO-$R^{18}$-CO-, und falls $R^1$-$Q^1$- oder $R^2$-$Q^2$- fehlen $R^{10}$, $R^{13}$, $R^{16}$ , Het-CO oder einen über eine -CONH-, -COO-, -NH-C(=S)-NH-, -NH-C(=O)-NH-, $-SO_2NH-$ oder -NHCO- Bindung verknüpften fluoreszierenden Farbstoffrest,

Z jeweils unabhängig voneinander einen Aminosäurerest oder einen Di-, Tri- oder Tetrapeptidrest, wobei die Aminosäuren unabhängig voneinander ausgewählt sind aus einer Gruppe bestehend aus Ala; Asn, Asp, Arg, Cys, Gln, Glu, Gly, His, Ile, Leu, Lys, Met, Phe, Pro, Ser, Thr, Trp, Tyr, Val oder M,
wobei die genannten Aminosäuren auch zu N-Methyl-, N-Ethyl-, N-Propyl-, N-Benzyl- oder $C_\alpha$-Methylderivaten derivatisiert sein können, und die Aminosäurereste über die $\alpha$-Amino- und $\alpha$-Carboxygruppen peptidartig miteinander verknüpft sind, und
wobei M immer enthalten ist,

M $NH(R^5)$-$CH(R^3)$-COOH,

$R^3$ $-R^5$-$R^4$, $-R^6$-$R^4$, $-R^7$-$R^4$,

$R^4$ OH, $NH_2$, SH oder COOH,

$R^5$ Alkylen mit 1-6 C-Atomen,

$R^6$ Alkylenphenylen mit 7-14 C-Atomen,

$R^7$ Alkylenphenylalkylen mit 8-15 C-Atomen,

$R^8$ H, A oder Alkylenphenyl mit 7-12 C-Atomen,

A Alkyl mit 1-6 C-Atomen,

$R^{10}$ einfach durch COOH, COOA, $SR^{11}$ oder $NR^{12}R^{12'}$ substituiertes Alkanoyl mit 1-18 C-Atomen,

$R^{11}$ Trityl, Pyridyl-2-thio oder Alkyl-thio mit 1-6 C-Atomen,

$R^{12}$ H, Alkyl mit 1-8 C-Atomen oder eine Aminoschulzgruppe,

$R^{12'}$ Alkyl mit 1-8 C-Atomen oder eine Amino-Schutzgruppe

$R^{13}$ unsubstituiertes oder ein- oder zweifach durch Alkyl mit 1-6 C-Atomen, Alkoxy mit 1-4 C-Atomen, Alkanoyl mit 1-8 C-Atomen, Hal, $SR^{14}$ oder $NR^{15}R^{15'}$ substituiertes Aroyl mit 7-11 C-Atomen,

$R^{14}$ H oder A,

$R^{15}$, $R^{15'}$ jeweils unabhängig voneinander H oder A,

$R^{16}$ unsubstituiertes oder im Arylteil ein-, zwei- oder dr fach durch Hal, Alkoxy mit 1-6 C-Atomen oder OH substituiertes Aralkanoyl mit 7-19 C-Atomen, worin Arylteil auch eine

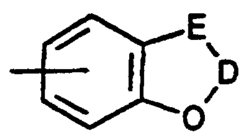

- Gruppe sein kann,

E $CH_2$ oder O,

D Carbonyl oder $[C(R^{17}R^{17'})]_m$,

$R^{17}$, $R^{17'}$ jeweils unabhängig voneinander H oder A,

$R^{18}$ fehlt oder $R^{19}$, $R^{20}$, $R^{19}$-$R^{20}$-$R^{19}$, unsubstituiertes oder ein- oder zweifach durch $R^5$ substituiertes Phenylen, wobei die Kettenlänge von $R^5$ jeweils unabhängig voneinander ist,

$R^{19}$ Alkylen mit 1-8 C-Atomen, wobei 1 oder 2 Methylen gruppen durch S, -CH=CH- oder -C≡C- ersetzt sein können,

$R^{20}$ Cycloalkylen mit 3-7 C-Atomen,

Hal F, Cl, Br oder I,

Het einen ein- oder zweikemigen gesättigten, ungesättigten oder aromatischen Heterocyclus mit 1 bis 4 N-, O- und/oder S-Atomen, über N oder C gebunden, der unsubstituiert oder ein-, zwei- oder dreifach durch Hal, A, $R^3$, $NR^4R^{4'}$, CN, $NO_2$-undloder Carbonylsauerstoff substituiert sein kann,

n 1, 2, 3, 4, 5, 6, 7, 8, 9 oder 10

und

m 1 oder 2 bedeuten,

wobei, sofern es sich um Reste optisch aktiver Aminosäuren und Aminosäurederivate handelt, sowohl die D- als auch die L-Formen eingeschlossen sind,
sowie deren Salze.

**2.** Verbindungen der Formel I gemäß Anspruch 1

$$R^1\text{-}Q^1\text{-}X\text{-}Q^2\text{-}R^2 \qquad I$$

c) worin

| | |
|---|---|
| $Q^1$, $Q^2$ und $R^2$ | fehlen, |
| $R^1$ | Cyclo-(Arg-Gly-Asp-D-Phe-Lys) und |
| X | Salicyloyl bedeuten; |

d) worin

| | |
|---|---|
| $Q^1$ und $Q^2$ | fehlen, |
| $R^1$ und $R^2$ | Cyclo-(Arg-Gly,Asp-D-Phe-Lys) und |
| X | $-CO-(CH_2)_2-CO-$ bedeuten; |

f) worin

| | |
|---|---|
| $Q^2$ und $R^2$ | fehlen, |
| $Q^1$ | $-NH-(CH_2)_5-CO-$, |
| $R^1$ | Cyclo-(Arg-Gly-Asp-D-Phe-Lys) und |
| X | Fluoreszeinoyl bedeuten; |

g) worin

| | |
|---|---|
| $Q^2$ und $R^2$ | fehlen, |
| $Q^1$ | $-NH-(CH_2)_5-CO-$, |
| $R^1$ | Cyclo-(Arg-Gly-Asp-D-Phe-Lys) und |
| X | tert-Butyloxycarbonyl bedeuten; |

sowie die physiologisch unbedenklichen Salze der genannten Verbindungen.

**3.** Verfahren zur Herstellung von Verbindungen der Formel I nach Anspruch 1 sowie ihrer Salze, **dadurch gekennzeichnet, daß** man

(a) eine Verbindung der Formel II

$$H\text{-}Q^1\text{-}R^1 \qquad II$$

worin
$Q^1$ und $R^1$ die in Anspruch 1 angegebene Bedeutung haben, in einer Acylierungsreaktion mit einer Verbindung der Formel III

$$X\text{-}L \qquad III$$

worin
X die in Anspruch 1 angegebene Bedeutung hat, und

L Cl, Br, I oder eine freie oder reaktionsfähig funktionell abgewandelte OH-Gruppe bedeutet, umsetzt

oder

b) daß man eine Verbindung der Formel IV

$$H\text{-}Q^2\text{-}R^2 \qquad IV$$

worin
$Q^2$ und $R^2$ die in Anspruch 1 angegebene Bedeutung haben, in einer Acylierungsreaktion mit einer Verbindung der Formel V

$$R^1\text{-}Q^1\text{-}X\text{-}L \qquad V$$

worin
$R^1$, $Q^1$, X und L die angegebene Bedeutung haben, umsetzt,
oder

c) daß man eine Verbindung der Formel II

$$H\text{-}Q^1\text{-}R^1 \qquad II$$

worin
$Q^1$ und $R^1$ die in Anspruch 1 angegebene Bedeutung haben,
in einer Additionsreaktion mit einer Verbindung der Formel VI

$$X\text{-}U \qquad VI$$

worin

X die in Anspruch 1 angegebene Bedeutung hat und
U -N=C=O, -N=C=S oder Maleinimidyl bedeutet, umsetzt,

oder

d) daß man sie aus einem ihrer funktionellen Derivate durch Behandeln mit einem solvolysierenden oder hydrogenolysierenden Mittel in Freiheit setzt,

und/oder daß man eine basische oder saure Verbindung der Formel I durch Behandeln mit einer Säure oder Base in eines ihrer Salze überführt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze als Integrininhibitoren zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

7. Verwendung von Verbindungen der Formel I nach Anspruch 1 und/oder ihre physiologisch unbedenklichen Salze zur Herstellung eines Arzneimittels.

**8.** Verwendung von Verbindungen der Formel I nach Anspruch 1 und/oder ihrer physiologisch unbedenklichen Salze bei der Bekämpfung von Krankheiten.

**9.** Verwendung von Verbindungen der Formel I nach Anspruch 1, 9. wobei X einen über eine -CONH-, -COO-, -NH-C(=S)-NH-, -NHwobei X einen über eine -CONH-, -COO-, -NH-C(=S)-NH-, -NH-C(=O)-NH-, $-SO_2NH-$ oder -NHCO-Bindung verknüpften fluoreszierenden Farbstoffrest bedeutet, als diagnostische Marker in der FACS-Analyse und Fluoreszenz-Mikroskopie.

**10.** Verwendung von Verbindungen der Formel I nach Anspruch 1 in der Affinitätschromatographie.

## Claims

**1.** Compounds of the formula I

$$R^1\text{-}Q^1\text{-}X\text{-}Q^2\text{-}R^2 \qquad I$$

in which

$Q^1$, $Q^2$ are each, independently of one another, absent or denote $-NH-(CH_2)_n-CO-$,

$R^1$, $R^2$ are each, independently of one another, absent or denote cyclo-(Arg-Gly-Asp-Z), where Z is bonded to $Q^1$ or $Q^2$ in the side chain or, if $Q^1$ and/or $Q^2$ is absent, is bonded to X, and

where at least one of the radicals $R^1$ or $R^2$ must always be present,

X denotes $-CO-R^{18}-CO-$, and, if $R^1$-$Q^1$- or $R^2$-$Q^2$- are absent, denotes $R^{10}$, $R^{13}$, $R^{16}$, Het-CO, or a fluorescent dye radical linked via a -CONH-, -COO-, -NH-C(=S)-NH-, -NH-C(=O)-NH-, $-SO_2NH-$ or -NHCO- bond,

Z, in each case independently of one another, denotes an amino acid radical or a di-, tri- or tetrapeptide radical, where the amino acids are selected, independently of one another, from a group consisting of Ala, Asn, Asp, Arg, Cys, Gln, Glu, Gly, His, Ile, Leu, Lys, Met, Phe, Pro, Ser, Thr, Trp, Tyr, Val or M,
where the said amino acids may also be derivatised to N-methyl, N-ethyl, N-propyl, N-benzyl or $C_\alpha$-methyl derivatives, and the amino acid radicals are linked to one another in a peptide-like manner via the $\alpha$-amino and $\alpha$-carboxyl groups, and
where M is always present,

M denotes $NH(R^8)-CH(R^3)-COOH$,

$R^3$ denotes $-R^5-R^4$, $-R^6-R^4$, $-R^7-R^4$,

$R^4$ denotes OH, $NH_2$, SH or COOH,

$R^5$ denotes alkylene having 1-6 C atoms,

$R^6$ denotes alkylenephenylene having 7-14 C atoms,

$R^7$ denotes alkylenephenylalkylene having 8-15 C atoms,

$R^8$ denotes H, A or alkylenephenyl having 7-12 C atoms,

A denotes alkyl having 1-6 C atoms,

$R^{10}$ denotes alkanoyl having 1-18 C atoms which is monosubstituted by COOH, COOA, $SR^{11}$ or

$NR^{12}R^{12'}$,

$R^{11}$ denotes trityl, pyridyl-2-thio or alkylthio having 1-6 C atoms,

$R^{12}$ denotes H, alkyl having 1-8 C atoms or an amino-protecting group,

$R^{12'}$ denotes alkyl having 1-8 C atoms or an amino-protecting group,

$R^{13}$ denotes aroyl having 7-11 C atoms which is unsubstituted or mono- or disubstituted by alkyl having 1-6 C atoms, alkoxy having 1-4 C atoms, alkanoyl having 1-8 C atoms, Hal, $SR^{14}$ or $NR^{15}R^{15'}$,

$R^{14}$ denotes H or A,

$R^{15}$, $R^{15'}$ each, independently of one another, denote H or A,

$R^{16}$ denotes aralkanoyl having 7-19 C atoms which is unsubstituted or mono-, di- or trisubstituted in the aryl moiety by Hal, alkoxy having 1-6 C atoms or OH and in which the aryl moiety may also be a

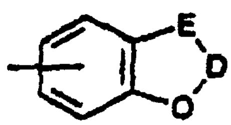

group,

E denotes $CH_2$ or O,

D denotes carbonyl or $[C(R^{17}R^{17'})]_m$,

$R^{17}$, $R^{17'}$ each, independently of one another, denote H or A,

$R^{18}$ is absent or
denotes $R^{19}$, $R^{20}$, $R^{19}$-$R^{20}$-$R^{19}$, phenylene which is unsubstituted or mono- or disubstituted by $R^5$, where the chain length of $R^5$ is in each case independent of one another,

$R^{19}$ denotes alkylene having 1-8 C atoms, in which 1 or 2 methylene groups may be replaced by S, -CH=CH- or -C≡C-,

$R^{20}$ denotes cycloalkylene having 3-7 C atoms,

Hal denotes F, Cl, Br or I,

Het denotes a mono- or bicyclic saturated, unsaturated or aromatic heterocyclic radical having 1 to 4 N, O and/or S atoms, bonded via N or C, which may be unsubstituted or mono-, di- or trisubstituted by Hal, A, $R^3$, $NR^4R^{4'}$, CN, $NO_2$ and/or carbonyl oxygen,

n denotes 1, 2, 3, 4, 5, 6, 7, 8, 9 or 10

and

m denotes 1 or 2,

where, if these are radicals of optically active amino acids and amino acid derivatives, both the D and the L forms are included,
and salts thereof.

**2.** Compounds of the formula I according to Claim 1

$$R^1\text{-}Q^1\text{-}X\text{-}Q^2\text{-}R^2 \qquad I$$

c) in which

| | |
|---|---|
| $Q^1$, $Q^2$ and $R^2$ | are absent, |
| $R^1$ | denotes cyclo-(Arg-Gly-Asp-D-Phe-Lys) and |
| X | denotes salicyloyl; |

d) in which

| | |
|---|---|
| $Q^1$ and $Q^2$ | are absent, |
| $R^1$ and $R^2$ | denote cyclo-(Arg-Gly-Asp-D-Phe-Lys) and |
| X | denotes $-CO-(CH_2)_2-CO-$; |

f) in which

| | |
|---|---|
| $Q^2$ and $R^2$ | are absent, |
| $Q^1$ | denotes $-NH-(CH_2)_5-CO-$, |
| $R^1$ | denotes cyclo-(Arg-Gly-Asp-D-Phe-Lys) and |
| X | denotes fluoresceinoyl; |

g) in which

| | |
|---|---|
| $Q^2$ and $R^2$ | are absent, |
| $Q^1$ | denotes $-NH-(CH_2)_5-CO-$, |
| $R^1$ | denotes cyclo-(Arg-Gly-Asp-D-Phe-Lys) and |
| X | denotes tert-butoxycarbonyl; |

and physiologically acceptable salts of the said compounds.

**3.** Process for the preparation of compounds of the formula I according to Claim 1 and salts thereof, **characterised in that**

(a) a compound of the formula II

$$H\text{-}Q^1\text{-}R^1 \qquad II$$

in which
$Q^1$ and $R^1$ have the meaning indicated in Claim 1, is reacted in an acylation reaction with a compound of the formula III
in which

$$X\text{-}L \qquad III$$

X has the meaning indicated in Claim 1, and
L denotes Cl, Br, I or a free or reactively functionally modified OH group,

or

b) **in that** a compound of the formula IV

$$H\text{-}Q^2\text{-}R^2 \qquad IV$$

in which
$Q^2$ and $R^2$ have the meaning indicated in Claim 1, is reacted in an acylation reaction with a compound of the formula V

$$R^1\text{-}Q^1\text{-}X\text{-}L \qquad V$$

in which
$R^1$, $Q^1$, X and L have the meaning indicated,
or

c) **in that** a compound of the formula II

$$H\text{-}Q^1\text{-}R^1 \qquad II$$

in which
$Q^1$ and $R^1$ have the meaning indicated in Claim 1,
is reacted in an addition reaction with a compound of the formula VI

$$X\text{-}U \qquad VI$$

in which

X has the meaning indicated in Claim 1 and
U denotes -N=C=O, -N=C=S or maleimidyl,

or

d) **in that** they are liberated from one of their functional derivatives by treatment with a solvolysing or hydrogenolysing agent,

and/or **in that** a basic or acidic compound of the formula I is converted into one of its salts by treatment with an acid or base.

4. Process for the preparation of pharmaceutical preparations, **characterised in that** a compound of the formula I according to Claim 1 and/or one of its physiologically acceptable salts is brought into a suitable dosage form together with at least one solid, liquid or semiliquid excipient or adjuvant.

5. Pharmaceutical preparation, **characterised by** a content of at least one compound of the formula I according to Claim 1 and/or one of its physiologically acceptable salts.

6. Compounds of the formula I according to Claim 1 and physiologically acceptable salts thereof as integrin inhibitors for combating pathologically angiogenic diseases, thromboses, cardiac infarction, coronary heart diseases, arteriosclerosis, tumours, osteoporosis, inflammations and infections.

7. Use of compounds of the formula I according to Claim 1 and/or physiologically acceptable salts thereof for the preparation of a medicament.

8. Use of compounds of the formula I according to Claim 1 and/or physiologically acceptable salts thereof in the combating of diseases.

9. Use of compounds of the formula I according to Claim 1 in which X denotes a fluorescent dye radical linked via a -CONH-, -COO-, -NH-C(=S)-NH-, -NH-C(=O)-NH-, $-SO_2NH-$ or -NHCO- bond, as diagnostic markers in FACS analysis and fluorescence microscopy.

**10.** Use of compounds of the formula I according to Claim 1 in affinity chromatography.

**Revendications**

**1.** Composés de formule 1

$$R^1\text{-}Q^1\text{-}X\text{-}Q^2\text{-}R^2 \qquad I$$

dans laquelle

$Q^1$, $Q^2$ sont chacun, indépendamment l'un de l'autre, absents ou désignent $-NH-(CH_2)_n-CO-$,

$R^1$, $R^2$ sont chacun, indépendamment l'un de l'autre, absents ou désignent cyclo-(Arg-Gly-Asp-Z), où Z est lié à $Q^1$ ou $Q^2$ dans la chaîne latérale ou, si $Q^1$ et/ou $Q^2$ est absent, est lié à X, et

où au moins l'un des radicaux $R^1$ ou $R^2$ doit toujours être présent,

X désigne $-CO-R^{18}-CO-$, et, si $R^1$-$Q^1$- ou $R^2$-$Q^2$- sont absents, désigne $R^{10}$, $R^{13}$, $R^{16}$, Hét-CO, ou un radical de colorant fluorescent lié par l'intermédiaire d'une liaison -CONH-, -COO-, -NH-C(=S) -NH-, -NH-C(=O)-NH-, $-SO_2NH-$ ou -NHCO-,

Z, dans chaque cas indépendamment l'un de l'autre, désigne un radical d'acide aminé ou un radical de di-, tri- ou tétrapeptide, où les acides aminés sont choisis, indépendamment les uns des autres, parmi un groupe constitué par Ala, Asn, Asp, Arg, Cys, Gln, Glu, Gly, His, Ile, Leu, Lys, Met, Phe, Pro, Ser, Thr, Trp, Tyr, Val ou M,
où lesdits acides aminés peuvent également être dérivés en dérivés N-méthylés, N-éthylés, N-propylés, N-benzylés ou $C_\alpha$-méthylés, et les radicaux d'acides aminés sont liés les uns aux autres d'une manière semblable à un peptide par l'intermédiaire des groupements α-amino et α-carboxyle, et
où M est toujours présent,

M désigne $NH(R^8)-CH(R^3)-COOH$,

$R^3$ désigne $-R^5-R^4$, $-R^6-R^4$, $-R^7-R^4$,

$R^4$ désigne OH, $NH_2$, SH ou COOH,

$R^5$ désigne alkylène ayant de 1 à 6 atomes de C,

$R^6$ désigne alkylènephénylène ayant de 7 à 14 atomes de C,

$R^7$ désigne alkylènephénylalkylène ayant de 8 à 15 atomes de C,

$R^8$ désigne H, A ou alkylènephényle ayant de 7 à 12 atomes de C,

A désigne alkyle ayant de 1 à 6 atomes de C,

$R^{10}$ désigne alkanoyle ayant de 1 à 18 atomes de C qui est monosubstitué par COOH, COOA, $SR^{11}$ ou $NR^{12}R^{12'}$,

$R^{11}$ désigne trityle, pyridyl-2-thio ou alkylthio ayant de 1 à 6 atomes de C,

$R^{12}$ désigne H, alkyle ayant de 1 à 8 atomes de C ou un groupement protecteur d'amino,

$R^{12'}$ désigne alkyle ayant de 1 à 8 atomes de C ou un groupement protecteur d'amino,

$R^{13}$ désigne aroyle ayant de 7 à 11 atomes de C qui est non substitué ou mono- ou disubstitué par alkyle ayant de 1 à 6 atomes de C, alcoxy ayant de 1 à 4 atomes de C, alcanoyle ayant de 1 à 8 atomes de C, Hal, $SR^{14}$ ou $NR^{15}R^{15'}$,

$R^{14}$ désigne H ou A,

$R^{15}$, $R^{15'}$ désignent chacun, indépendamment l'un de l'autre, H ou A,

$R^{16}$ désigne aralkanoyle ayant de 7 à 19 atomes de C qui est non substitué ou mono-, di- ou trisubstitué dans le motif aryle par Hal, alcoxy ayant de 1 à 6 atomes de C ou OH et dans lequel le motif aryle peut également être un groupement

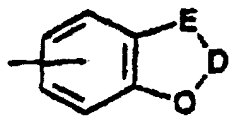,

E désigne $CH_2$ ou O,

D désigne carbonyle ou $[C(R^{17}R^{17'})]_m$,

$R^{17}$, $R^{17'}$ désignent chacun, indépendamment l'un de l'autre, H ou A,

$R^{18}$ est absent ou
désigne $R^{19}$, $R^{20}$, $R^{19}$-$R^{20}$-$R^{19}$, phénylène qui est non substitué ou mono- ou disubstitué par $R^5$, où la longueur de chaîne de $R^5$ est dans chaque cas indépendante l'une de l'autre,

$R^{19}$ désigne alkylène ayant de 1 à 8 atomes de C, dans lequel 1 ou 2 groupements méthylène peuvent être remplacés par S, -CH=CH- ou -C≡C-,

$R^{20}$ désigne cycloalkylène ayant de 3 à 7 atomes de C,

Hal désigne F, Cl, Br ou I,

Hét désigne un radical hétérocyclique mono- ou bicyclique saturé, insaturé ou aromatique ayant de 1 à 4 atomes de N, O et/ou S, lié par l'intermédiaire de N ou de C, qui peut être non substitué ou mono-, di- ou trisubstitué par Hal, A, $R^3$, $NR^4R^{4'}$, CN, $NO_2$ et/ou oxygène carbonylé,

n désigne 1, 2, 3, 4, 5, 6, 7, 8, 9 ou 10

et

m désigne 1 ou 2,

où, si ceux-ci sont des radicaux d'acides aminés et de dérivés d'acides aminés optiquement actifs, les formes tant D que L sont incluses,
et les sels de ceux-ci.

**2.** Composés de formule I selon la revendication 1

$$R^1\text{-}Q^1\text{-}X\text{-}Q^2\text{-}R^2 \qquad \text{I}$$

c) dans laquelle

$Q^1$, $Q^2$ et $R^2$ sont absents,
$R^1$ désigne cyclo-(Arg-Gly-Asp-D-Phe-Lys) et
X désigne salicyloyle ;

d) dans laquelle

$Q^1$ et $Q^2$ sont absents,
$R^1$ et $R^2$ désignent cyclo-(Arg-Gly-Asp-D-Phe-Lys) et
X désigne $-CO-(CH_2)_2-CO-$ ;

f) dans laquelle

$Q^2$ et $R^2$ sont absents,
$Q^1$ désigne $-NH-(CH_2)_5-CO-$,
$R^1$ désigne cyclo-(Arg-Gly-Asp-D-Phe-Lys) et
X désigne fluorescéinoyle ;

g) dans laquelle

$Q^2$ et $R^2$ sont absents,
$Q^1$ désigne $-NH-(CH_2)_5-CO-$,
$R^1$ désigne cyclo-(Arg-Gly-Asp-D-Phe-Lys) et
X désigne tertio-butoxycarbonyle ;

et les sels physiologiquement acceptables desdits composés.

**3.** Procédé de préparation de composés de formule I selon la revendication 1 et de sels de ceux-ci, **caractérisé en ce que**

(a) un composé de formule II

$$H-Q^1-R^1 \qquad II$$

dans laquelle
$Q^1$ et $R^1$ ont la signification indiquée selon la revendication 1, est réagi dans une réaction d'acylation avec un composé de formule III
dans laquelle

$$X-L \qquad III$$

X a la signification indiquée selon la revendication 1, et
L désigne Cl, Br, I ou un groupement OH libre ou modifié de manière réactive et fonctionnelle,

ou

b) **en ce qu'**un composé de formule IV

$$H-Q^2-R^2 \qquad IV$$

dans laquelle
$Q^2$ et $R^2$ ont la signification indiquée selon la revendication 1, est réagi dans une réaction d'acylation avec un composé de formule V

$$R^1\text{-}Q^1\text{-}X\text{-}L \qquad V$$

dans laquelle
$R^1$, $Q^1$, X et L ont la signification indiquée,
ou

c) **en ce qu'**un composé de formule II

$$H\text{-}Q^1\text{-}R^1 \qquad II$$

dans laquelle
$Q^1$ et $R^1$ ont la signification indiquée selon la revendication 1,
est réagi dans une réaction d'addition avec un composé de formule VI

$$X\text{-}U \qquad VI$$

dans laquelle

X a la signification indiquée selon la revendication 1 et
U désigne -N=C=O, -N=C=S ou maléimidyle,

ou

d) **en ce qu'**ils sont libérés depuis l'un de leurs dérivés fonctionnels par un traitement par un agent de solvolysation ou d'hydrogénolyse,

et/ou **en ce qu'**un composé basique ou acide de formule I est converti en l'un de ses sels par un traitement par un acide ou par une base.

4. Procédé de préparation de préparations pharmaceutiques, **caractérisé en ce qu'**un composé de formule I selon la revendication 1 et/ou one l'un de ses sels physiologiquement acceptables est mis sous une forme de dosage convenable conjointement avec au moins un excipient ou adjuvant solide, liquide ou semi-liquide.

5. Préparation pharmaceutique, **caractérisée par** une teneur en au moins un composé de formule I selon la revendication 1 et/ou en l'un de ses sels physiologiquement acceptables.

6. Composés de formate I selon la revendication 1 et les sels physiologiquement acceptables de ceux-ci comme inhibiteurs de l'intégrine pour lutter contre les maladies pathologiquement angiogéniques, les thromboses, l'infarctus du myocarde, les maladies coronariennes, l'artériosclérose, les tumeurs, l'ostéoporose, les inflammations et les infections.

7. Utilisation de composés de formule I selon la revendication 1 et/ou de sels physiologiquement acceptables de ceux-ci pour la préparation d'un médicament.

8. Utilisation de composés de formule I selon la revendication 1 et/ou de sels physiologiquement acceptables de ceux-ci pour lutter contre des maladies.

9. Utilisation de composés de formule I selon la revendication 1 dans laquelle X désigne un radical de colorant fluorescent lié par l'intermédiaire d'une liaison -CONH-, -COO-, -NH-C(=S)-NH-, -NH-C(=O)-NH-, $-SO_2NH-$ ou -NHCO-,
en tant que marqueurs de diagnostic en analyse par FACS et en microscopie à fluorescence.

10. Utilisation de composés de formule I selon la revendication 1 en chromatographie d'affinité.